# EUROPEAN PATENT APPLICATION

(11) **EP 1 345 152 A2**
(43) Date of publication of application: **17.09.2003**
(21) Application number: 02025421.5
(22) Date of filing: 14.11.2002
(51) Int. Cl.: G06F 19/00

(54) **Medical-information supplying method and apparatus**

(30) Priority: 12.03.2002 JP 2002066563
(71) Applicant: Colin Corporation, Komaki-shi, Aichi-ken (JP)
(72) Inventor: Oka, Tohru, Komaki-shi, Aichi-ken (JP); Narimatsu, Kiyoyuki, Komaki-shi, Aichi-ken (JP)
(74) Representative: Winter, Brandl, Fürniss, Hübner, Röss, Kaiser, Polte Partnerschaft

(57) **Abstract**

A method of supplying, from a server which is connected via a communication line to a plurality of member's terminal devices which are respectively operated by a plurality of members who are registered in advance in the server, a set of medical information stored in the server, to one of the member's terminal devices that is operated by one of the members, the method including a medical-information supplying step of supplying, based on a set of physical information of the one member inputted by the one member's terminal device, the set of medical information stored in the server and related to the set of physical information of the one member, to the one member's terminal device, a physical-information storing step of storing, in the server, respective sets of physical information of the members inputted by the member's terminal devices, and an outputting step of outputting, when a pass code prepared for the one member is inputted by a user's terminal device which is operated by a user and which is connected via the communication line to the server, the set of physical information of the one member stored in the server and corresponding to the pass code, to the user's terminal device.

## Description

### BACKGROUND OF THE INVENTION

### Field of the Invention

The present invention relates to a medical-information supplying method and a medical-information supplying apparatus that are used by an individual member for medical consultation or physical examination and allows the physical information inputted and stored therefor to be used for the individual member, thereby avoiding a repetitive diagnosis.

### Related Art Statement

When a person enters a company or a school, takes a qualifying examination, or buys a life insurance, it is needed to accurately know a physical condition of the person. To this end, the person has conventionally been required to submit a medical certificate. Meanwhile, when a person visits a medical institution for inspecting a disease, in particular, when the person first visits the institution, the institution cannot readily obtain the record of past physical information of the person and must make a diagnosis based on only the current physical information obtained from the person.

However, it is cumbersome for a person who enters a company or a school, takes a qualifying examination, or buys a life insurance to visit a medical institution, undergo a medical examination, and obtain a medical certificate. In addition, this costs the expense of some money or time. Furthermore, when a person consults a medical institution, the institution makes a diagnosis based on only the current physical information obtained from the person, and accordingly the accuracy of the diagnosis made may not be satisfactorily high.

### SUMMARY OF THE INVENTION

It is therefore an object of the present invention to provide a medical-information supplying method and a medical-information supplying apparatus that allow the physical information stored for an individual person, to be easily utilized by a third party without needing to apply an additional load, such as re-examination, to the person.

The above object has been achieved by the present invention. According to a first aspect of the present invention, there is provided a method of supplying, from a server which is connected via a communication line to a plurality of member's terminal devices which are respectively operated by a plurality of members who are registered in advance in the server, a set of medical information stored in the server, to one of the member's terminal devices that is operated by one of the members, the method comprising a medical-information supplying step of supplying, based on a set of physical information of the one member inputted by the one member's terminal device, the set of medical information stored in the server and related to the set of physical information of the one member, to the one member's terminal device, a physical-information storing step of storing, in the server, respective sets of physical information of the members inputted by the member's terminal devices, and an outputting step of outputting, when a pass code prepared for the one member is inputted by a user's terminal device which is operated by a user and which is connected via the communication line to the server, the set of physical information of the one member stored in the server and corresponding to the pass code, to the user's terminal device.

According to this aspect, when the user inputs the pass code prepared for the individual member only, the physical information related to the member and stored in the server, is outputted to the user's terminal device through which the pass code has been inputted. Therefore, for example, when a person enters a corporation or a school, takes a qualifying examination, or buys a life insurance, the corporation, the school, an organization that administrates the qualifying examination, or an insurance company can easily obtain, using the pass code permitted by the person, his or her past physical information stored in the server and check his or her physical condition. Thus, those parties need not require the person to obtain a medical certificate from a medical institution. In addition, regarding an outpatient who consults a medical institution such as a hospital, the medical institution can easily obtain, using a pass code permitted by the outpatient, his or her past physical information stored in the server and make a diagnosis based on the thus obtained physical information. This diagnosis can enjoy a higher reliability.

According to a preferred feature of the first aspect of the present invention, the medical-information supplying step comprises making a diagnosis about a physical condition of the one member based on the set of physical information of the one member inputted by the one member's terminal device, and supplying the set of medical information including the made diagnosis, to the one member's terminal device.

According to this feature, the set of medical information supplied to the individual member includes not only the physical information of the member but also the diagnosis made based on the physical information. Therefore, the member can more accurately recognize his or her physical condition and can obtain the reliable diagnosis.

According to another feature of the first aspect of the present invention, the medical-information supplying method further comprises a charging step of charging, when the set of physical information of the one member stored in the server is outputted to the user's terminal device, the user of the user's terminal device for use of the set of physical information of the one member.

According to this feature, a medical-information supplying company that owns the server and supplies the medical information or the physical information stored in the server can easily manage its business. In addition, the fee paid by each individual member can be reduced or zeroed, which leads to increasing the total number of the individual members who are registered in the server.

According to a second aspect of the present invention, there is provided a medical-information supplying apparatus including a server which is connected via a communication line to a plurality of member's terminal devices which are respectively operated by a plurality of members who are registered in advance in the server, and supplying a set of medical information stored in the server, to one of the member's terminal devices that is operated by one of the members, the apparatus comprising a medical-information supplying means for supplying, based on a set of physical information of the one member inputted by the one member's terminal device, the set of medical information stored in the server and related to the set of physical information of the one member, to the one member's terminal device; a physical-information storing means for storing, in the server, respective sets of physical information of the members inputted by the member's terminal devices; and an outputting means for outputting, when a pass code prepared for the one member is inputted by a user's terminal device which is operated by a user and which is connected via the communication line to the server, the set of physical information of the one member stored in the server and corresponding to the pass code, to the user's terminal device.

According to this aspect, when the user inputs the pass code prepared for the individual member only, the outputting means outputs the physical information related to the member and stored in the server, to the user's terminal device through which the pass code has been inputted. Therefore, for example, when a person enters a corporation or a school, takes a qualifying examination, or buys a life insurance, the corporation, the school, an organization that administrates the qualifying examination, or an insurance company can easily obtain, using the pass code permitted by the person, his or her past physical information stored in the server and check his or her physical condition. Thus, those parties need not require the person to obtain a medical certificate from a medical institution. In addition, regarding an outpatient who consults a medical institution such as a hospital, the medical institution can easily obtain, using a pass code permitted by the outpatient, his or her past physical information stored in the server and make a diagnosis based on the thus obtained physical information. This diagnosis can enjoy a higher reliability.

According to a preferred feature of the second aspect of the present invention, the medical-information supplying means comprises a diagnosing means for making a diagnosis about a physical condition of the one member based on the set of physical information of the one member inputted by the one member's terminal device, and means for supplying the set of medical information including the made diagnosis, to the one member's terminal device.

According to this feature, the set of medical information supplied to the individual member includes not only the physical information of the member but also the diagnosis made based on the physical information. Therefore, the member can more accurately recognize his or her physical condition and can obtain the reliable diagnosis.

According to another feature of the second aspect of the present invention, the medical-information supplying apparatus further comprises a charging means for charging, when the set of physical information of the one member stored in the server is outputted to the user's terminal device, the user of the user's terminal device for use of the set of physical information of the one member.

According to this feature, a medical-information supplying company that owns the server and supplies the medical information or the physical information stored in the server can easily manage its business. In addition, the fee paid by each individual member can be reduced or zeroed, which leads to increasing the total number of the individual members who are registered in the server.

According to another feature of the second aspect of the present invention, the medical-information supplying apparatus further comprises a plurality of physical-information obtaining devices which are connected to the member's terminal devices and which obtain the respective sets of physical information and supply the respective sets of physical information to the member's terminal devices.

According to this feature, the members need not input manually the respective sets of physical information into the respective member's terminal devices. In addition, a diagnosis made based on each set of physical information can enjoy a high reliability.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a medical-information supplying system as a medical-information supplying apparatus utilizing a communication line, to which the present invention is applied;
Fig. 2 is a block diagram for explaining essential control functions of a server shown in Fig. 1;
Fig. 3 is a view showing an example of a set of medical information that is supplied from the server of Fig. 1 to an individual member;
Fig. 4 is a view showing an example of a set of medical information about an individual member that is supplied to a dealer who has made access to the server of Fig. 1;
Fig. 5 is a flow chart representing a portion of the essential control functions of the server of Fig. 1, i.e., a virtual-hospital controlling routine; and
Fig. 6 is a flow chart representing another portion of the essential control functions of the server of Fig. 1, i.e., an individual-medical-information-supply controlling routine.

### DETAILED DESCRIPTION OF PREFERRED EMBODIMENTS

Hereinafter, there will be described a preferred embodiment of the present invention in detail by reference to the drawings.

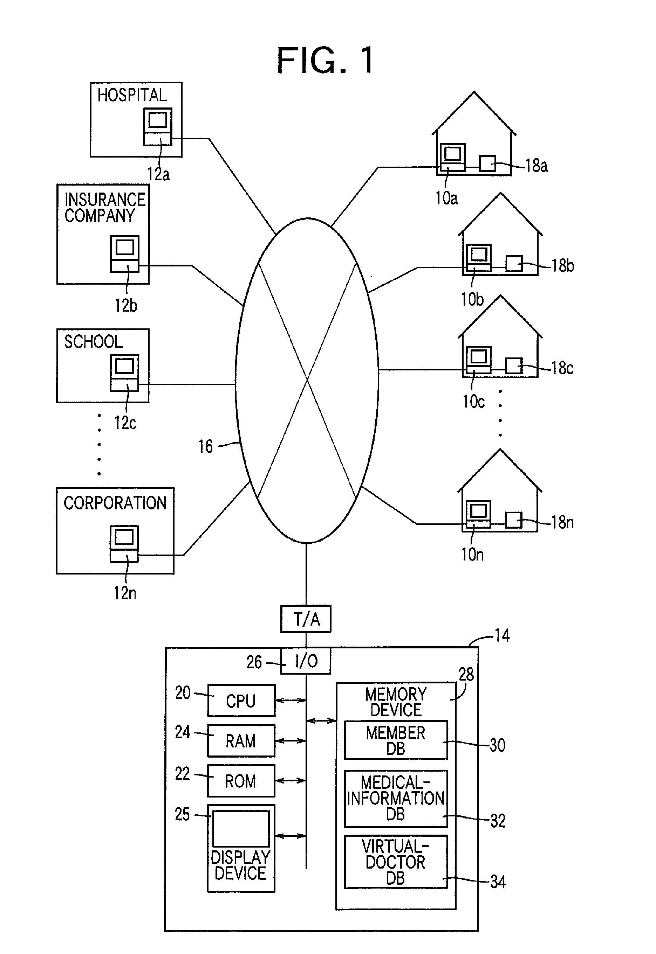

Fig. 1 is a view for explaining the construction of a medical-information supplying system as a medical-information supplying apparatus to which the present invention is applied. As shown in Fig. 1, the present medical-information supplying system includes a plurality of stationary-type or portable-type member's terminal devices 10a, 10b, 10c, ..., 10n that are operable by respective individual members. The member's terminal devices may be television sets, personal computers, or portable telephones that have the function of making communications via the internet. The supplying system additionally includes a plurality of user's terminal devices 12a, 12b, 12c, ..., 12n that are operable by respective users, such as hospitals, insurance companies, schools, or corporations; and a server 14 as a virtual hospital that is provided in an information supplying company. The supplying system further includes a communication line 16, such as wire or wireless internet (i.e., communication network) or wire or wireless telephone line, connects the member's terminal devices 10n, the user's terminal devices 12n, and the server 14, with one another, so that highly secret codes, such as SSL, can be communicated among those elements 10n, 12n, 14. The member's terminal devices 10n and the user's (i.e., dealer's) terminal devices 12n may be provided by, e.g., personal computers including display devices, and the server 14 may be provided by, e.g., a high-speed and high-capacity electronic computer.

A plurality of physical-information obtaining devices 18a, 18b, 18c, ..., 18n are connected to the plurality of member's terminal devices 10a, 10b, 10c, ..., 10n, respectively. Each of the physical-information obtaining devices 18 periodically obtains, from a corresponding one of the individual members (i.e., individual living persons), various sorts of physical information including blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes. Each of the physical-information obtaining devices 18 may include a plurality of sensors that detect the above-indicated various sorts of physical information, or an input device that is operable by a person to input the above-indicated various sorts of physical information. The physical-information obtaining devices 18n supply the thus obtained respective sets of physical information to the member's terminal devices 10n, respectively. The blood pressure BP, e.g., systolic and diastolic blood pressure values, may be detected in such a manner that each of the physical-information obtaining devices 18n employs an inflatable cuff, the cuff is wound around an upper arm of the corresponding living person, and a blood pressure BP of the person is determined, according to so-called oscillometric method, based on change of respective amplitudes of respective heartbeat-synchronous pulses of a cuff pulse wave detected by a pressure sensor as a pressure signal from the cuff during changing of the pressing pressure of the cuff. Alternatively, the blood pressure BP may be obtained in such a manner that blood pressure values measured using a common sphygmomanometer are inputted through operation of keys of an input device. The weight W may be detected in such a manner that each of the physical-information obtaining devices 18n employs a weight sensor, and a weight of the corresponding living person is detected by the weight sensor, or alternatively the weight W may be obtained in such a manner that a weight measured using a common scales is inputted through operation of keys of an input device. The heart rate HR may be calculated based on the number of the heartbeat-synchronous pulses of the above-described cuff pulse wave that are produced in unit time, or based on the number of heartbeat-synchronous pulses of an electrocardiograph waveform that are produced in unit time and are detected by an electrocardiograph device, not shown. The electrocardiograph waveform ECG may be detected by an electrocardiograph device, not shown. The temperature TB may be detected by a temperature sensor, not shown, that is supported by the above-described cuff and is worn with the cuff on each living person. The autonomic-nerve activity may be determined based on fluctuations of the heart rate values HR or the blood pressure values BP. The pulse-wave propagation velocity PWV may be determined based on a propagation time from the time when a second heart sound II is detected by a heart-sound microphone to the time when a dicrotic notch of a carotid pulse wave is detected, and a propagation distance between the heart and the carotid artery. The augmentation index AI may be defined as a ratio or proportion of one of an amplitude of an incident-wave component of an arterial pulse wave, such as carotid pulse wave, and an amplitude of a reflected-wave component of the pulse wave, to the other, and may be determined based on the waveform of the carotid pulse wave or the cuff pulse wave.

The server 14 is essentially provided by a so-called microcomputer including a CPU (central processing unit) 20, a ROM (read only memory) 22 that stores control programs, a RAM (random access memory) 24 that functions as a temporary-storage device, a display device 25, and an input-and-output interface 26 that is connected via a terminal adaptor TA to the communication line 16. The server 14 includes a memory device 28 that stores various sorts of data bases (DB) corresponding to various sorts of information. More specifically described, the memory device 28 includes a member DB (data base) 30 that stores respective names of a plurality of living persons who have contracted, at their own charge or no charge, with the information supplying company to become, in advance, individual members who are supplied with medical information from the company; respective identification codes ID of the individual members; and respective pass codes or words of the individual members. The memory device 28 additionally includes a medical-information DB (data base) 32 that stores medical information including the respective sets of physical information sent from the individual members, and respective medical evaluations or diagnoses made based on those sets of physical information. The memory device 28 further includes a virtual-doctor DB (data base) 34 that stores a diagnosing program for use in making those medical evaluations or diagnoses based on the sets of physical information or respective changes of the sets of information.

Thus, the member's terminal devices 10n are operated by the respective individual members who have been registered in the server 14, and each one of the member's terminal devices 10n periodically sends a set of physical information of a corresponding one of the individual members to the server 14. At that time, the server 14 stores the set of physical information, and automatically makes, according to the diagnosing program pre-stored in the virtual-doctor DB 34, a medical evaluation or diagnosis based on the set of physical information or a change or trend of the set of physical information. In addition, the server 14 sends the thus made medical evaluation or diagnosis, together with the current and past sets of physical information, to the each member's terminal device 10n, so that the medical evaluation or diagnosis and the current and past sets of physical information are displayed on the terminal device 10n. Meanwhile, each one of the user's terminal devices 12n is operated by a corresponding one of the users who uses the pass code permitted by each one of the individual members to request the server 14 to send the medical information related to the each individual member. At that time, the server 14 reads, from the sets of medical information stored in the medical-information DB 32, the medical information related to the individual member corresponding to the pass code, and sends the thus obtained medical information to the each user's terminal device 12n, so that the medical information is displayed on the terminal device 12n.

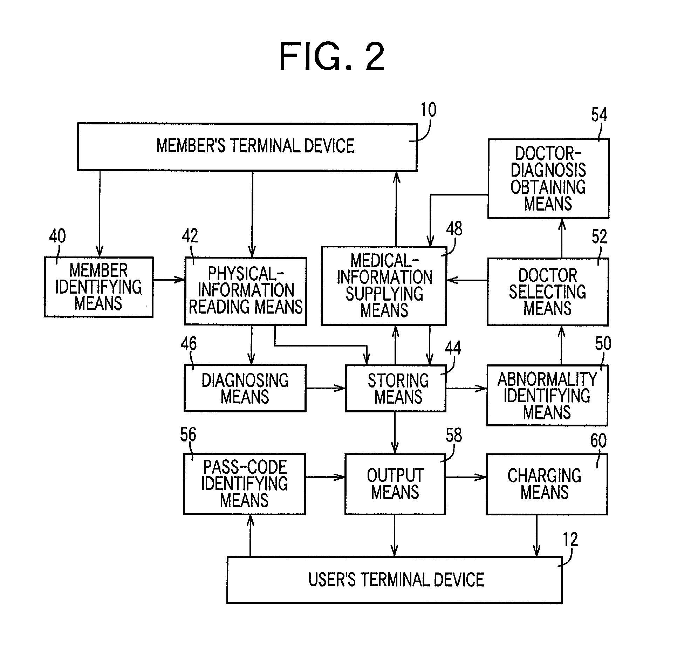

Fig. 2 is a block diagram for explaining the essential control functions of the above-described server 14. A member identifying means 40 judges whether an identification code ID inputted and sent by an arbitrary one of the member's terminal devices 10n is identical with one of the identification codes pre-stored in the member DB 30, and thereby judges whether a person who has made access to the server 14 is one of the individual members pre-registered in the server 14. A physical-information reading means 42 reads, when the member identifying means 40 has identified the person as one of the individual members, the set of physical information sent by the member's terminal device 10n, and a storing means 44 stores the thus read set of physical information in such a manner that the set of physical information is associated with the identification code ID of the individual member. A diagnosing means 46 automatically makes a diagnosis based on the set of physical information read by the reading means 42, according to the diagnosing program pre-stored in the virtual-doctor DB 34. For example, the diagnosing means 46 judges whether the set of physical information read by the reading means 42 falls in a predetermined reference range and thereby makes a medical evaluation or diagnosis about the set of physical information. The storing means 44 stores the thus made medical evaluation or diagnosis such that the medical evaluation or diagnosis is associated with the set of physical information or the identification code ID. A medical-information supplying means 48 supplies, to the member's terminal device 10n that has sent the current set of physical information, the current and past sets of physical information, and the medical evaluation or diagnosis made on those sets of physical information, all of which have been stored by the storing means 44, so that the trend of physical information and the medical evaluation or diagnosis are displayed on the terminal device 10n.

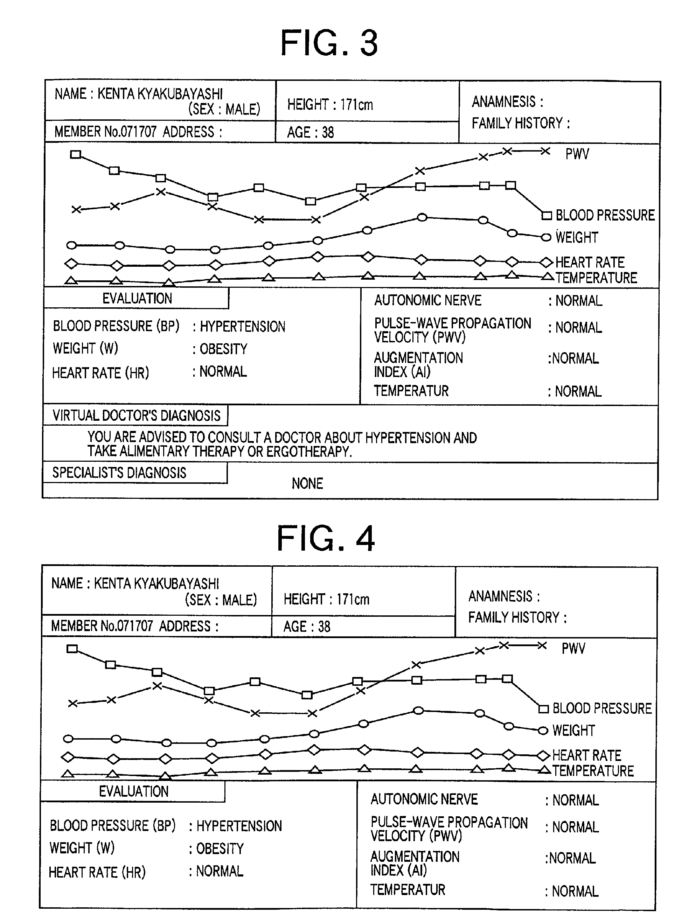

An abnormality identifying means 50 judges whether each set of physical information read by the reading means 42 falls outside a predetermined normal range and thereby judges whether each individual member is abnormal. The normal range may be identical with the above-described reference range. A doctor selecting means 52 selects, when the abnormality identifying means 50 judges based on the set of physical information that the individual member is abnormal, an appropriate one of a list of doctors pre-stored in the memory device 28, based on the sort of the physical information judged as abnormal, and an address of the individual member, such that the selected doctor is specialized in the sort of the abnormality and is near to the address of the individual member. A doctor-diagnosis obtaining means 54 sends the physical information judged as abnormal, to the appropriate doctor selected by the doctor selecting means 52, requests the doctor to make a diagnosis about the set of physical information, and receives the diagnosis made by the doctor. The storing means 44 stores the diagnosis made by the doctor, such that the diagnosis is associated with the identification code ID or the physical information, and the medical-information supplying means 48 sends the diagnosis made by the doctor, to the individual member. Fig. 3 shows an example of a set of medical information that is displayed on a display device of the member's terminal device 10n that has sent the set of physical information to the server 14.

A pass code identifying means 56 judges whether the member's name and the pass code that have been inputted through one of the user's terminal devices 12n by one of the users pre-registered in the server 14, are identical with respective ones of the member's names and the pass codes pre-stored (i.e., pre-registered) in the memory device 28. If a positive judgment is made, the identifying means 56 judges that the user's terminal device 12n is being operated by the user permitted by one of the individual members. The use of the pass code is limited to designated times (e.g., only one time), or a designated date, that is registered, in advance, in the server 14 by the individual member. An outputting means 58 outputs (i.e., sends), when the identifying means 56 judges that the use of the pass code has been allowed by the individual member, a set of medical information that has been stored in the medical-information DB 32 by the storing means 44 and is associated with the individual member who has allowed the use of the pass code, to the user's terminal device 12n that has made access to the server 14. Fig. 14 shows an example of the set of medical information that is displayed by the user's terminal device 12n. A charging means 60 charges the user of the user's terminal device 12n that has made access to the server 14, for the use of the medical information that has been stored for the individual member. More specifically described, the charging means 60 produces data to issue a bill and sends the data to the user's terminal device 12n so that the data are displayed or outputted by the terminal device 12n.

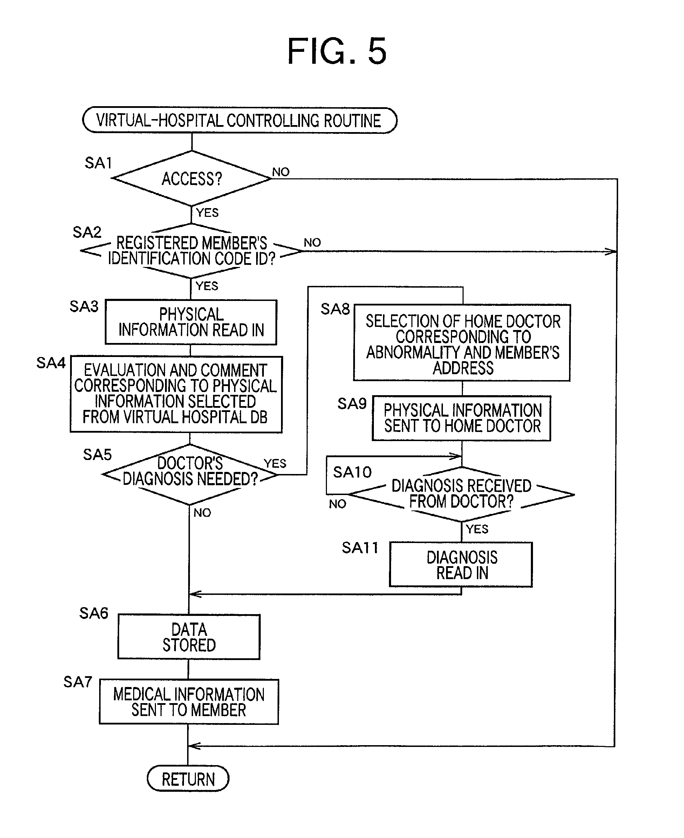

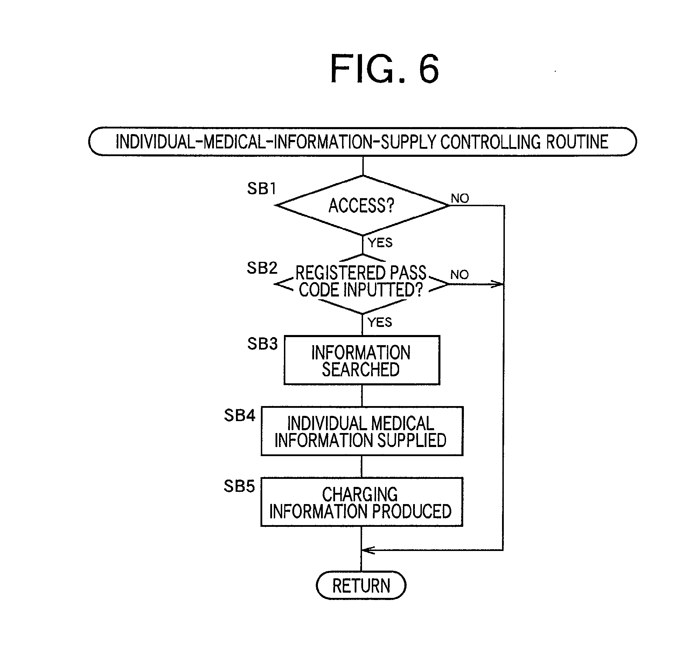

Figs. 5 and 6 show flow charts representing the essential control functions of the above-described server 14, more specifically described, Fig. 5 shows a virtual-hospital controlling routine, and Fig. 6 shows an individual-medical-information-supply controlling routine. Each of the routines shown in Figs. 5 and 6 is iteratively executed at a short period.

At Step SA1 of Fig. 5 (hereinafter, "Step(s)" is omitted), the server 14 judges whether any one of the member's terminal devices 10n has made access to the server 14. If a negative judgment is made at SA1, this routine is quitted. On the other hand, if a positive judgment is made at SA1, the control goes to SA2 corresponding to the member identifying step or the member identifying means 40. At SA2, the server judges whether the member's identification code ID inputted by the member's terminal device 10n is identical with one of the member's identification codes ID registered in advance in the server. If a negative judgment is made at SA2, this routine is quitted. On the other hand, if a positive judgment is made at SA 2, the control goes to SA3 corresponding to the physical-information reading step or the physical-information reading means 42. At SA3, the server reads at least one set of physical information that has been obtained by the physical-information obtaining device 18n of the member's terminal device 10n and has been sent from the terminal device 10n, i.e., at least one of blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes.

Then, the control goes to SA4 corresponding to the diagnosing step or the diagnosing means 46. At SA4, the server automatically makes, according to the automatically evaluating and diagnosing program stored in the virtual-doctor DB 34, a diagnosis about the new set of physical information, the past set or sets of physical information, and a change of the new and past sets of physical information. For example, the server selects one of a plurality of evaluations or comments that corresponds to the new set of physical information. Fig. 3 shows the thus made evaluation and the virtual doctor's diagnosis. Then, the control goes to SA5 corresponding to the abnormality identifying step or the abnormality identifying means 50. At SA5, the server judges whether it is needed to consult an actual doctor or a specialist. More specifically described, the server judges whether the new set of physical information falls outside a predetermined normal rage. If a negative judgment is made at SA5, the control goes to SA6 corresponding to the storing step or the storing means 44. At SA6, the server stores, in the medical-information DB 32, the new set of physical information and the automatically made evaluation or diagnosis, such that those medical information is associated with the identification code ID of the individual member. Then, the control goes to SA7 corresponding to the medical-information supplying step or the medical-information supplying means 48. At SA7, the server sends, to the member's terminal device 10n that has sent the new set of physical information, a set of medical information of the individual member that has been stored in the medical-information DB 32, that is, the new and past sets of physical information (i.e., the trend of physical information) and the automatic evaluation or diagnosis, so that the set of medical information is displayed on the member's terminal device 10n as shown in Fig. 3. Thus, each individual member can easily and economically request the virtual hospital or the virtual doctor to check his or her physical condition or make a diagnosis on it.

On the other hand, if a positive judgment is made at SA5, the control goes to SA8 corresponding to the doctor selecting step or the doctor selecting means 52. At SA8, the server selects, from a pre-stored list of doctors, a doctor or a specialist who is appropriate for the sort of the abnormal physical information, the degree of abnormality of the physical information, and the address of the individual member. Next, the control goes to SA 9 to SA11 corresponding to the doctor-diagnosis obtaining step or the doctor-diagnosis obtaining means 54. First, at SA9, the server sends, to the selected doctor, the abnormal, new set of physical information of the individual member and the past sets of physical information of the same, and requests the doctor to make a diagnosis on the sets of physical information. Then, at SA10, the server judges whether the server has received a diagnosis sent from the doctor. SA10 is repeated till a positive judgment is made. When a positive judgment is made at SA10, the control goes to SA11 to read in the diagnosis sent from the doctor. Then, the control goes to SA6 and SA7. At SA6, the server stores, in the medical-information DB 32, not only the new set of physical information and the automatically made evaluation or diagnosis, but also the diagnosis made by the actual doctor, such that those medical information is associated with the identification code ID of the individual member. Then, the control goes to SA7 corresponding to the medical-information supplying step or the medical-information supplying means 48. At SA7, the server sends, to the member's terminal device 10n that has sent the new set of physical information, a set of medical information of the individual member that has been stored in the medical-information DB 32, that is, the new and past sets of physical information (i.e., the trend of physical information), the automatic evaluation or diagnosis, and the diagnosis made by the actual doctor, so that the set of medical information is displayed on the member's terminal device 10n, as shown in Fig. 3, and additionally the diagnosis made by the actual doctor, and the name and address of the hospital to which the doctor belongs, or the address of the doctor are indicated in an area prepared therefor (i.e., the lowermost area). Thus, each individual member can easily and economically request the virtual hospital or the virtual doctor to check his or her physical condition or make a diagnosis on it, and additionally can obtain the diagnosis made by the actual doctor when the new set of physical information is judged as abnormal. If necessary, the individual member can request the doctor to re-examine his or her physical condition.

In Fig. 6, at SB1, the server 14 judges whether any one of the respective user's terminal devices 12n of the users pre-registered in the server has made access to the server. If a negative judgment is made at SB1, this routine is quitted. On the other hand, if a positive judgment is made at SB1, the control goes to SB2 corresponding to the pass-code identifying step or the pass-code identifying means 56. At SB2, the server judges whether the pass code inputted by the user's terminal device 12n is identical with the pass code pre-registered in the server. If a negative judgment is made at SB2, this routine is quitted. On the other hand, if a positive judgment is made at SB2, the control goes to SB3 to search, in the medical-information DB 32, for the medical information related to the individual member who has permitted the use of the pass code. Then, the control goes to SB4 corresponding to the outputting step or the outputting means 58. At SB4, the medical information related to the individual member, obtained from the medical-information DB 32, is sent to the user's terminal device 12n that has made access to the server 14, so that the medical information is displayed on the terminal device 12n, as shown in Fig. 4. Next, the control goes to SB5 corresponding to the charging step or the charging means 60. At SB5, the server produces charging information to charge the user's terminal device 12n, that is, the user who has made access, using the pass code given by the individual member, to the server to obtain the medical information related to the member. At a step, not shown, the server automatically issues, based on the charging information, a bill to charge the above-indicated user. The amount of charging and the address to which the bill is sent may be determined in a contract made between the server and the user, or may be noticed to, or inputted by, the user on the display of the user's terminal device 12n.

As is apparent from the foregoing description of the illustrated embodiment, when a user such as a corporation, a school, an insurance company, or a hospital inputs a pass code that is pre-registered in the server 14 by an individual member and is given by the member to the user, the server 14 outputs the physical information related to the member, stored at SA6 (the physical-information storing step or the physical-information storing means 44), to the user's terminal device 12n through which the pass code has been inputted. Therefore, for example, when a person enters a corporation or a school, takes a qualifying examination, or buys a life insurance, the corporation, the school, an organization that administrates the qualifying examination, or an insurance company can easily obtain, using the pass code permitted by the person, his or her past physical information stored in the server and check his or her physical condition. Thus, those parties need not require the person to obtain a medical certificate from a medical institution. In addition, regarding an outpatient who consults a medical institution such as a hospital, the medical institution can easily obtain, using a pass code permitted by the outpatient, his or her past physical information stored in the server and make a diagnosis based on the thus obtained physical information. This diagnosis can enjoy a higher reliability.

In addition, in the illustrated embodiment, the server 14 automatically makes, at SA4 (the diagnosing step or the diagnosing means 46), a diagnosis about the physical condition of the individual member based on the new set of physical information inputted through one of the member's terminal devices 10n, the past sets of physical information related to the individual member, and the change of the new and past sets of physical information, and supplies, at SA7 (the medical- information supplying step or the medical-information supplying means 48), the medical information including the diagnosis automatically made at SA4 (the diagnosing step or the diagnosing means 46). Thus, the medical information supplied to the individual member includes not only the current and past sets of physical information of the member but also the diagnosis made based on those sets of physical information. Therefore, the individual member can more accurately recognize his or her physical condition and can obtain the reliable diagnosis.

Moreover, in the illustrated embodiment, when the server 14 outputs, at SB4 (the outputting step or the outputting means 58), the sets of physical information related to the individual member and stored in the server, the server charges, at SB5 (the charging step or the charging means 60), the user of the user's terminal device 12n that receives the sets of physical information outputted from the server. Therefore, the medical-information supplying company that owns the server 14 and supplies the physical information or the medical information stored in the server 14 can easily manage its business. In addition, the fee paid by each individual member can be reduced or zeroed, which leads to increasing the total number of the individual members.

While the present invention has been described in its embodiment by reference to the drawings, it is to be understood that the present invention can otherwise be embodied.

For example, in the illustrated embodiment, the server 14 is explained such that the server 14 is provided by a single computer. However, the server 14 may be provided by a plurality of computers, and those computers may be provided at respective positions remote from each other.

In the illustrated embodiment, each of the physical-information obtaining devices 18n connected to the member's terminal devices 10n has the functions of obtaining blood pressure BP, weight W, heart rate HR, electrocardiograph waveform ECG, temperature TB, autonomic-nerve activity, pulse-wave propagation velocity PWV, augmentation index AI, etc. However, each physical-information obtaining device 18n may be one that can obtain only a portion (one, two, ..., but not all) of the above-indicated sorts of physical information, or one that can obtain other sorts of physical information. The sorts of physical information that are not directly detected by each physical-information obtaining device 18n may be manually inputted through each member's terminal device 10n after having been measured using scales, a sphygmomanometer, a heart-rate meter, an electrocardiograph, etc. In this sense, each physical-information obtaining device 18n may be one that does not include any sensors.

In the flow charts shown in Figs. 5 and 6, the order of the steps may be changed, as needed. In addition, SA5 to SA11 of Fig. 5 may be omitted.

In the illustrated embodiment, the user who is registered in advance in the server 14 can obtain the physical information of the individual member, stored in the server 14. However, the illustrated system may be modified such that a user who is not registered in advance, that is, a dealer can obtain the physical information of the individual member, stored in the server 14.

While the present invention has been described in detail in its embodiments by reference to the drawings, it is to be understood that the present invention is not limited to the details of the described embodiments but may be embodied with various changes or improvements that may occur to a person skilled in the art.

## Claims

1. A method of supplying, from a server which is connected via a communication line to a plurality of member's terminal devices which are respectively operated by a plurality of members who are registered in advance in the server, a set of medical information stored in the server, to one of the member's terminal devices that is operated by one of the members, the method comprising:
a medical-information supplying step of supplying, based on a set of physical information of said one member inputted by said one member's terminal device, the set of medical information stored in the server and related to the set of physical information of said one member, to said one member's terminal device,
a physical-information storing step of storing, in the server, respective sets of physical information of the members inputted by the member's terminal devices, and
an outputting step of outputting, when a pass code prepared for said one member is inputted by a user's terminal device which is operated by a user and which is connected via the communication line to the server, the set of physical information of said one member stored in the server and corresponding to the pass code, to the user's terminal device.

2. A method according to claim 1, wherein the medical-information supplying step comprises making a diagnosis about a physical condition of said one member based on the set of physical information of said one member inputted by said one member's terminal device, and supplying the set of medical information including the made diagnosis, to said one member's terminal device.

3. A method according to claim 1 or claim 2, further comprising a charging step of charging, when the set of physical information of said one member stored in the server is outputted to the user's terminal device, the user of the user's terminal device for use of the set of physical information of said one member.

4. A medical-information supplying apparatus including a server (14) which is connected via a communication line (16) to a plurality of member's terminal devices (10) which are respectively operated by a plurality of members who are registered in advance in the server, and supplying a set of medical information stored in the server, to one (10n) of the member's terminal devices that is operated by one of the members, the apparatus comprising:
a medical-information supplying means (46, 48, 50, 52, 54) for supplying, based on a set of physical information of said one member inputted by said one member's terminal device (10n), the set of medical information stored in the server (14) and related to the set of physical information of said one member, to said one member's terminal device;
a physical-information storing means (40, 42, 44) for storing, in the server (14), respective sets of physical information of the members inputted by the member's terminal devices (10); and
an outputting means (56, 58) for outputting, when a pass code prepared for said one member is inputted by a user's terminal device (12) which is operated by a user and which is connected via the communication line (16) to the server (14), the set of physical information of said one member stored in the server and corresponding to the pass code, to the user's terminal device (12).

5. An apparatus according to claim 4, wherein the medical-information supplying means (46, 48, 50, 52, 54) comprises a diagnosing means (46) for making a diagnosis about a physical condition of said one member based on the set of physical information of said one member inputted by said one member's terminal device, and means (48) for supplying the set of medical information including the made diagnosis, to said one member's terminal device.

6. An apparatus according to claim 4 or claim 5, further comprising a charging means (60) for charging, when the set of physical information of said one member stored in the server (14) is outputted to the user's terminal device (12), the user of the user's terminal device for use of the set of physical information of said one member.

7. An apparatus according to any of claims 4 to 6, further comprising a plurality of physical-information obtaining devices (18) which are respectively connected to the member's terminal devices (10) and which obtain the respective sets of physical information and supply the respective sets of physical information to the member's terminal devices.
